# EUROPEAN PATENT SPECIFICATION

(11) **EP 2 235 215 B1**
(45) Date of publication and mention of the grant of the patent: **20.02.2013**
(21) Application number: 08870148.7
(22) Date of filing: 12.12.2008
(51) Int. Cl.: C12Q 1/68

(54) **NON-INVASIVE DETECTION OF BLADDER CANCER BY FLUORESCENCE IN SITU HYBRIDIZATION OF AURORA A**
NICHTINVASIVER NACHWEIS VON BLASENKREBS MITTELS FLUORESZENZ BEI IN-SITU-HYBRIDISIERUNG VON AURORA A
DÉTECTION NON INVASIVE DU CANCER DE LA VESSIE PAR HYBRIDATION FLUORESCENTE IN SITU D'AURORA A

(30) Priority: 03.01.2008 US 18730 P
(43) Date of publication of application: 06.10.2010
(73) Proprietor: Board of Regents, The University of Texas System, Austin, TX 78701 (US)
(72) Inventor: GROSSMAN, H. Barton, Bellaire TX 77401 (US); CZERNIAK, Bogdan, Houston TX 77079 (US); SUBRATA, Sen, Sugar Land, TX 77479 (US)
(74) Representative: Gowshall, Jonathan Vallance
(86) International application number: PCT/US2008/013632
(87) International publication number: WO 2009/088421

(56) References cited:
- STEINBERG JORDAN R ET AL: "Aurora A as a potential biomarker for bladder cancer" JOURNAL OF UROLOGY, BALTIMORE, MD, US, vol. 173, no. 4, suppl, 1 April 2005 (2005-04-01), page 166, XP009113863 ISSN: 0022-5347
- JEONG J ET AL: "Amplification of a mitotic kinase gene as a marker of bladder carcinoma." MODERN PATHOLOGY, vol. 16, no. 1, January 2003 (2003-01), page 155A, XP008054954 & 92ND ANNUAL MEETING OF THE UNITED STATES AND CANADIAN ACADEMY OF PATHOLOGY; WASHINGTON, D.C., USA; MARCH 22-28, 2003 ISSN: 0893-3952
- SPIESS PHILIPPE E ET AL: "Aurora A test for bladder cancer" PROCEEDINGS OF THE ANNUAL MEETING OF THE AMERICAN ASSOCIATION FOR CANCER RESEARCH, NEW YORK, NY, vol. 47, 1 January 2006 (2006-01-01), page 1061, XP001539403 ISSN: 0197-016X
- PARK HONG-SEOK ET AL: "Quantitation of Aurora kinase A gene copy number in urine sediments and bladder cancer detection." JOURNAL OF THE NATIONAL CANCER INSTITUTE 1 OCT 2008, vol. 100, no. 19, 1 October 2008 (2008-10-01), pages 1401-1411, XP009113892 ISSN: 1460-2105
- PARK H S ET AL: "Aurora kinase A as a biomarker for the detection of bladder cancer" MODERN PATHOLOGY, vol. 21, no. Suppl. 1, January 2008 (2008-01), page 176A, XP009113865 & 97TH ANNUAL MEETING OF THE UNITED-STATES-AND-CANADIAN-ACADEMY-OF-PATH OLOGY; DENVER, CO, USA; MARCH 01 -07, 2008 ISSN: 0893-3952
- SEN SUBRATA ET AL: "Amplification/overexpression of a mitotic kinase gene in human bladder cancer." JOURNAL OF THE NATIONAL CANCER INSTITUTE 4 SEP 2002, vol. 94, no. 17, 4 September 2002 (2002-09-04), pages 1320-1329, XP009113887 ISSN: 0027-8874 cited in the application
- BAFFA R ET AL: "Molecular genetics of bladder cancer: targets for diagnosis and therapy" JOURNAL OF EXPERIMENTAL AND CLINICAL CANCER RESEARCH, ROME, IT, vol. 25, no. 2, 1 June 2006 (2006-06-01), pages 145-160, XP009113870 ISSN: 0392-9078
- COMPÉRAT E ET AL: "Aurora-A/STK-15 is a predictive factor for recurrent behaviour in non-invasive bladder carcinoma: a study of 128 cases of non-invasive neoplasms" VIRCHOWS ARCHIV, SPRINGER, BERLIN, DE, vol. 450, no. 4, 28 February 2007 (2007-02-28), pages 419-424, XP019491849 ISSN: 1432-2307
- S BIESTERFELD ET AL: 'Polyploidy in non-neoplastic tissues' J CLIN PATHOL, [Online] 01 January 1994, pages 38 - 42, XP055011921 Retrieved from the Internet: <URL:http://www.ncbi.nlm.nih.gov/pmc/articl es/PMC501754/pdf/jclinpath00214-0040.pdf> [retrieved on 2011-11-11]
- RODNEY T MILLER: 'Using fluorescent in situ hybridization (UroVysion FISH) for monitoring of recurrence of urothelial carcinoma' PROPATH, [Online] 01 June 2004, XP055011922 Retrieved from the Internet: <URL:http://www.ihcworld.com/_newsletter/20 04/focus_jun_2004.pdf> [retrieved on 2011-11-11]
- 'AURKA (20q13) / Alphasatellite 20 DNA Probe, Dual-Colour, Direct-Labelled', [Online] 03 November 2011, XP055011153 Retrieved from the Internet: <URL:http://www.strettonscientific.co.uk/do wnloads/Q-BIOgeneApplicationManuals/PONC201 3_application_Manual_GB_printer.pdf> [retrieved on 2011-11-03]
- 'Poseidon(TM) Repeat Free(TM) AURKA (20q13) & 20q11 probe', [Online] 01 August 2008, XP055011148 Retrieved from the Internet: <URL:http://www.bioportugal.pt/imgs/produto s/5575_AM-KBI-10721_D1 0.pdf> [retrieved on 2011-11-03]
- 'Vysis AURKA SpectrumGold FISH Probe Kit' 03 November 2011, XP055011162

## Description

### BACKGROUND OF THE INVENTION

This invention was made with United States government support under grant number U01 CA 85078 awarded by the National Cancer Institute. The United States government has certain rights in the invention.

The present invention relates generally to the field of cancer detection. More particularly, it concerns the detection of bladder cancer.

### SUMMARY OF THE INVENTION

The present invention, as defined in claim 1, relates to a method of detecting bladder cancer in a patient, comprising isolating bladder cells from the patient's urine; hybridizing the bladder cells with a labelled DNA probe, wherein the probe recognizes at least a portion of the aurora kinase A gene, to yield a sample of bladder cells hybridized with the labelled DNA probe; and counting the number of bladder cells in the sample, the number of copies of the aurora kinase A gene in each bladder cell in the sample, and the number of bladder cells in the sample having at least a first threshold number of copies of the aurora kinase A gene, further comprising, if greater than or equal to a first threshold percentage of the bladder cells have at least the first threshold number of copies of the aurora kinase A gene, presuming the patient to have bladder cancer, wherein the first threshold number of copies of the aurora kinase A gene is three, and wherein the first threshold percentage is 15%.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

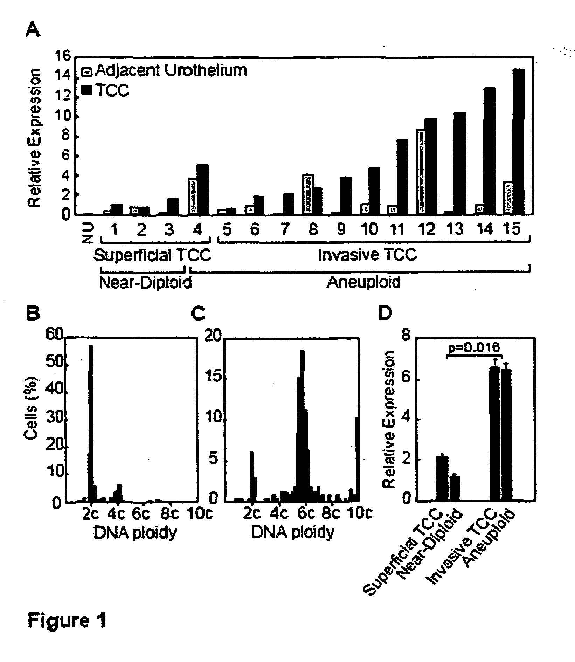

Figure 1. Expression of aurora kinase A in transitional cell carcinoma (TCC) and its relation to DNA ploidy. A, Levels of aurora kinase A in paired samples of adjacent urothelium and TCC revealed by quantitative RT-PCR. *B and C*. DNA histograms generated by image analysis of tumor nuclei showing near-diploid (2c) DNA content and pronounced aneuploidy (6c), respectively. D, Mean expression levels of aurora kinase A in low-grade (grade 1-2), superficial (Ta-Tla) and high-grade (grade 3), invasive (Tlb and higher) TCCs as well as near-diploid and aneuploid TCCs.

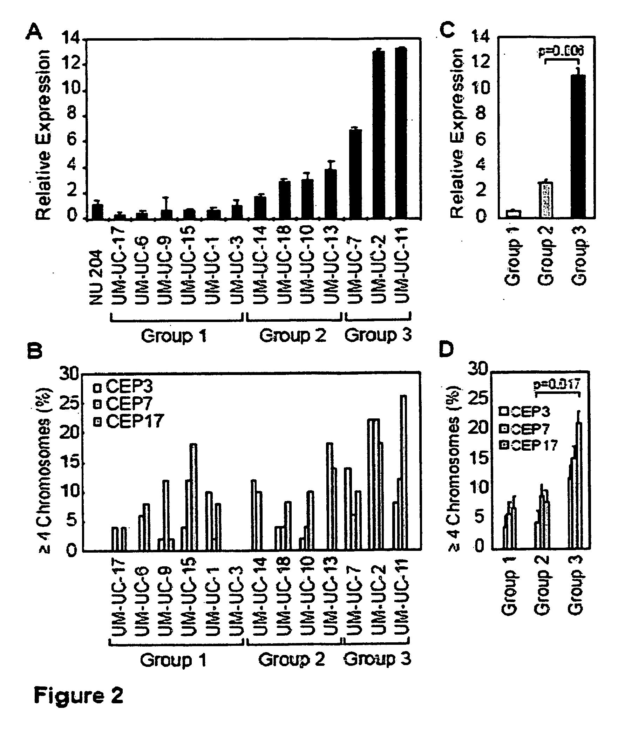

Figure 2. Aurora kinase A expression and chromosomal copy number in bladder cancer cell lines. A, Expression of aurora kinase A in bladder cancer cell lines by quantitative RT-PCR compared to cultured urothelial cells (NU204). B, Chromosomal copy number revealed by quantitative FISH using centromeric probes to chromosomes 3, 7, and 17. C, Mean expression levels of aurora kinase A in three groups of bladder cancer cell lines. D, Mean chrosomomal copy number in cancer cell lines revealed by FISH with centromeric probes to chromosomes 3, 7, and 17 in 3 groups of bladder cancer cell lines shown in C.

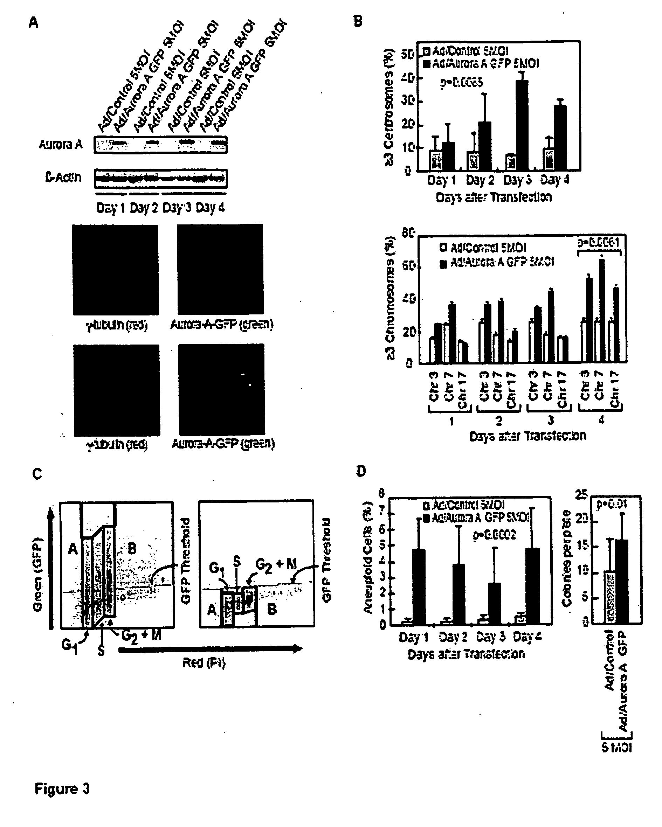

Figure 3. Ectopic expression of aurora kinase A-GFP fusion protein in SV40 urothelial cells transfected with adenoviral vector containing wild type aurora kinase A insert using anti-GFP antibody. A, Western blot analysis showing expression of aurora kinase A-GFP fusion protein *(upper panel).* Immunofluorescence analysis showing ectopic expression of aurora kinase A-GFP fusion protein with DAPI as a nuclear counterstain *(lower panel).* Immunofluorescence images of cells infected with an adenoviral vector without aurora kinase A insert *(a, b).* Image obtained with a red filter reveals two centrosomes (a). Image obtained with a green filter reveals no ectopic expression of aurora kinase A-GFP fusion protein (b). Immunofluorescence images of cells infected with an adenoviral vector containing aurora kinase A insert (c, d). Image obtained with a red filter shows multiplication of centrosomes revealed with anti-ytubulin antibody (red) (c). Image obtained with a green filter shows expression of ectopically expressed aurora kinase A-GFP protein in centrosomes (d). *B*, Quantitative assessment of centrosomes and chromosomal copy number induced by ectopically expressed aurora kinase A. C, Dual fluorescence FACS-analysis of ectopically expressed aurora kinase A-GFP protein (green fluorescence) in relation to DNA content revealed with PI counterstain (red fluorescence). Dual fluorescence scattergram after infection with adenoviral vector containing wild type aurora kinase A insert *(right panel).* Control scattergram of cells infected with empty adenoviral vector *(right panel).* Note increase of aneuploid cells in scattergram plot areas designated A and B *(right panel)* as compared to the control *(left panel). D,* Quantitative assessment of aneuploid cells revealed by dual fluorescence FACS analysis shown in C and colony formation on soft agar. Ad/Control: cells infected with an adenoviral vector without aurora kinase A insert, Ad/aurora kinase A GFP: cells transfected with adenoviral vector containing wild type Aurora kinase A, MOI: multiplicity of infection.

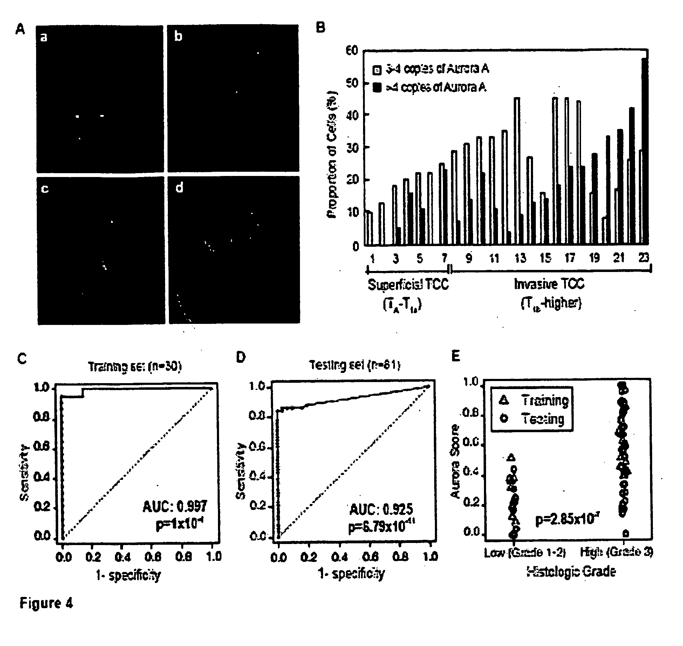

Figure 4. Detection of cancer cells in voided urine with aurora kinase A FISH probe. A, Dual fluorescence FISH demonstrating. aurora kinase A gene copy number (red) and short arm of chromosome specific probe (green). Diploid copy number of aurora kinase A in normal urothelial cells (a). Low-level of aurora kinase A gene amplification (3 copies) in a low-grade (grade 1-2) TCC (b). High-level of aurora kinase A gene amplification (>4 copies) in high-grade (grade 3) invasive TCC (c). Polysomy of chromosome 20 in a high-grade (grade 3) invasive TCC containing multiple copies of aurora kinase A and chromosome 20p probes (d). *B*, Quantitative FISH analysis of aurora kinase A gene copy number in voided urine specimens from 23 patients with TCC of the bladder (Training set). The percentages of cells showing low (3-4 copies) and high (> 4 copies) amplification levels of aurora kinase A in the individual patients are shown. C, ROC curve for aurora kinase A FISH test in training set consisting of 23 urine samples from patients with bladder cancer and seven urine samples from seven healthy unaffected controls (n=30). D, ROC curve for aurora kinase A FISH test in testing set consisting of 51 urine samples from patients with bladder cancer and 30 urine samples from healthy unaffected controls (n=81). E, aurora kinase A score by histologic grade.

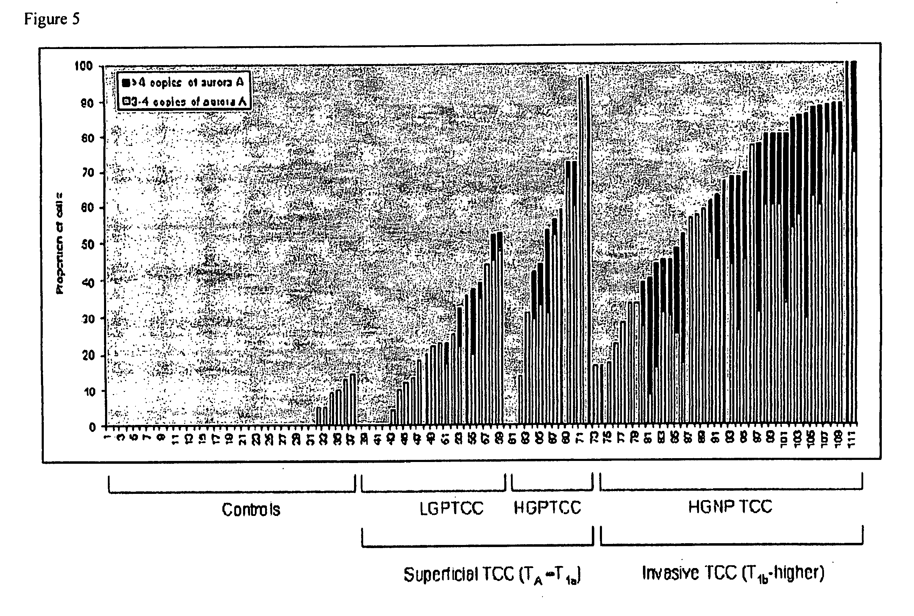

Figure 5. Quantitative FISH analysis of aurora kinase A gene copy number in voided urine samples from 74 patients with TCC of the bladder and 37 healthy controls (combined training and testing sets). The percentages of cells showing low (3-4 copies) and high (>4 copies) amplification levels of aurora kinase A in individual patients are shown.

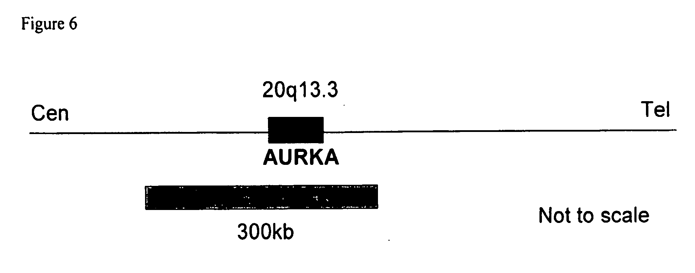

Figure 6. A map of the AURKA 20q13 probe.

### DESCRIPTION OF ILLUSTRATIVE EMBODIMENTS

Malignant cells, manifesting chromosomal instability in the form of aneuploidy, frequently show supernumerary centrosomes and multipolar mitotic spindles suggesting that abnormalities in organelles controlling segregation of chromosomes at mitosis play a role in the development of aneuploidy.¹⁻³

Aurora kinase A, also known as STK15 or BTAK, is a member of the serine threonine kinase family that includes *Drosophilia* aurora and *Saccharomyces cervisiae* JPL1 kinase.⁴⁻⁶ These kinases have been identified as critical regulators of normal chromosome segregation and centrosome functions including regulation of centrosome separation and maturation in human cells.⁷ Moreover, overexpression of the aurora kinase A gene, located on chromosome 20q13, has been shown to be associated with aneuploidy and chromosomal instability promoting tumorigenic transformation and progression in mammalian cells and in several human tumors including urothelial carcinoma.^{4,8}

Interestingly, primary human urothelial cells induced to undergo transformation *in vitro* manifest amplification of chromosome 20q13.2 suggesting that overexpression of gene(s) on this chromosome may play a significant role in the development of bladder cancer.⁹ These observations together with the facts that the aurora kinase A encoding gene on chromosome 20q13 has also been identified as a tumor susceptibility locus in mice and humans¹⁰ and aurora kinase A induces functional inactivation of p53 tumor suppressor gene⁵ imply that increased expression of aurora kinase A may be a critical genetic determinant of chromosomal instability and transformation in human urothelial cells.

In one embodiment, the present invention relates to a method of detecting bladder cancer in a patient, comprising isolating bladder cells from the patient's urine; hybridizing the bladder cells with a labelled DNA probe, wherein the probe recognizes at least a portion of the aurora kinase A gene, to yield a sample of bladder cells hybridized with the labelled DNA probe; and counting the number of bladder cells in the sample, the number of copies of the aurora kinase A gene in each bladder cell in the sample, and the number of bladder cells in the sample having at least a first threshold number of copies of the aurora kinase A gene.

Isolating bladder cells (most commonly, urothelial cells) from the patient's urine can be performed by any technique known in the art. In one embodiment, isolating bladder cells can be performed by collecting voided urine specimens (approximately 100-200 ml) from the patient, centrifuging at a rotational velocity and for a duration sufficient to pellet bladder cells from the urine, discarding the supernatant, and resuspending the pelleted bladder cells in an appropriate solution for storage or use. In one embodiment, the duration and rotational velocity of centrifugation are about 15 minutes at about 1000 rpm. In one embodiment, the pelleted bladder cells are resuspended in 2 ml of DMEM (Dulbecco/Vogt Modified Eagle's Minimal Essential Medium) with 10% of DMSO (dimethyl sulfoxide) and stored in -70°C until the time comes to thaw the resuspended bladder cells prior to their use in later steps of the method.

After thawing and prior to hybridization, the bladder cells can be washed one or more times, such as three times, in PBS (phosphate-buffered saline) or another buffer suitable for washing bladder cells, followed by centrifugation to pellet the washed bladder cells. The pelleted washed bladder cells can be fixed in methanol/acetic acid (3:1), pretreated in 2x SSC/0.5% NP-40, pH 7 at 37°C for 30 minutes, followed by dehydration in ethanol at 90°C for 5 minutes. As will be apparent to the person of ordinary skill in the art, other processing techniques suitable for preparing bladder cells for hybridization with a labelled DNA probe can be performed as a matter of routine experimentation.

Hybridizing the bladder cells with a labelled DNA probe can be performed by any technique known in the art. The labelled DNA probe can comprise any DNA sequence and any detectable moiety, provided the probe recognizes at least a portion of the aurora kinase A gene. In one embodiment, the detectable moiety comprises a fluorophore. In one embodiment, the detectable moiety comprises a radioisotope.

A cDNA sequence of aurora kinase A from *H. sapiens* is shown as SEQ ID NO: I (GenBank accession no. BC001280) and a genomic sequence of aurora kinase A from *H*. *sapiens* is shown as SEQ ID NO:2. Though otherwise identical, the two sequences differ at position 301 and the cDNA contains a 16mer adenosine tail which the genomic sequence lacks.

By "the probe recognizes at least a portion of the aurora kinase A gene" is meant that the probe comprises a DNA molecule having a sequence that is essentially fully complementary (*i.e.,* at least about 90% complementary, such as at least about 95% complementary, at least about 96% complementary, at least about 97% complementary, at least about 98% complementary, at least about 99% complementary, at least about 99.5% complementary, or at least about 99.9% complementary) to the sequence of at least one DNA strand of the portion of the aurora kinase A gene, and further, that the DNA molecule of the probe is both long enough and has a sequence such that it will essentially only hybridize to the aurora kinase A gene and will essentially not hybridize to any other gene or non-expressed genomic element of the bladder cell. It is further desirable that the DNA molecule of the probe is not so long as to require a long time to hybridize to the aurora kinase A gene. In one embodiment, the labelled DNA probe comprises AURKA 20q13 (MP Biomedicals/Qbiogene, Illkirch, France). AURKA 20q13 specific DNA probe is optimized to detect copy numbers of the Aurora kinase A gene on chromosome 20 at region 20q13 on metaphase/interphase spreads, blood smears and paraffin embedded tissue sections.

Any fluorophore which can be bound to the DNA molecule of the probe can be used. In one embodiment, the fluorophore is selected from the group consisting of rhodamine and fluorescein. In one embodiment, the AURKA 20q13 probe is directly labeled with Rhodamine (red) and the chromosome 20 α-satellite probe is directly labeled with Fluorescein (green). This dual color aurora kinase A FISH probe can be used to detect the copy number of the aurora kinase A gene in exfoliated cells of voided urine sediments.

In one embodiment, the hybridization technique can involve incubation of dehydrated bladder cells with 10 µl of AURKA 20q13 probe at 37°C for about 16 hr (*e.g*., overnight). To remove unhybridized probe, the cells can be washed, such as in 0.5x SSC/0.1% SDS for five minutes at 65°C. Cells can be counterstained with DAPI (4',6-diamidino-2-phenylindole) and mounted in an antifade solution prior to counting.

In a further embodiment, a second labelled DNA probe that is not specific for the aurora kinase A gene can be hybridized to the bladder cells to allow counting of other DNA regions that may be of interest to the person of ordinary skill in the art.

The hybridizing step yields a sample of bladder cells hybridized with the labelled DNA probe.

Counting the number of bladder cells in the sample, the number of copies of the aurora kinase A gene in each bladder cell in the sample, and the number of bladder cells in the sample having at least a first threshold number of copies of the aurora kinase A gene can be performed by any appropriate technique known to the person of ordinary skill in the art of flow cytometry, fluorescence microscopy, or radioisotopic detection. The use of flow cytometers or fluorescence-activated cell sorters is well known in the art. When the detectable moiety is a fluorophore, fluorescence signals can be counted and captured with any appropriate microscope, such as a Zeiss Axioplan 2 (Carl Zeiss MicroImaging GmbH, Jena, Germany).

Counting yields the number of bladder cells in the sample, the number of copies of the aurora kinase A gene in each bladder cell in the sample, and the number of bladder cells in the sample having at least a first threshold number of copies of the aurora kinase A gene. The first threshold number of copies can be chosen to be equal to the minimum number of copies of the aurora kinase A gene commonly found in bladder cancer cells. In one embodiment, the first threshold number of copies of the aurora kinase A gene is three.

In a further embodiment, counting further comprises counting the number of bladder cells having at least a second threshold number of copies of the aurora kinase A gene, wherein the second threshold number of copies is greater than the first threshold number of copies. The second threshold number of copies can be chosen to be equal to the minimum number of copies of the aurora kinase A gene commonly found in cells of aggressive bladder cancer. In one embodiment, the second threshold number of copies of the aurora kinase A gene is five.

By counting the number of bladder cells in the sample and the number of bladder cells in the sample having at least a first threshold number of copies of the aurora kinase A gene, the latter number can be divided by the number of bladder cells to calculate a percentage of the bladder cells that have at least a first threshold number of copies of the aurora kinase A

The method of the present invention further comprises, if greater than or equal to a first threshold percentage of the bladder cells have at least the first threshold number of copies of the aurora kinase A gene, the step of presuming the patient to have bladder cancer. Any value of the percentage of the bladder cells that have at least a first threshold number of copies of the aurora kinase A gene can be used as the first threshold percentage. In the present invention, the first threshold percentage is 15%.

In a further embodiment, the method further comprises, if greater than or equal to a second threshold percentage of the bladder cells have at least the first threshold number of copies of the aurora kinase A gene, wherein the second threshold percentage is greater than the first threshold percentage, the step of presuming the patient to have aggressive bladder cancer. "Aggressive" bladder cancer refers to bladder cancer characterized by one or more of presenting an invasive tumor, a nonpapillary tumor, or a tumor having histologic grade 3. In one embodiment, the first threshold percentage is 15% and the second threshold percentage is 20%.

By performing the method, factors useful in presuming whether the patient has bladder cancer and, if so, how aggressive, can be quantified by a physician by a technique that is noninvasive. By quantifying the factors, the physician can decide whether other diagnostic or therapeutic methods, such as cystoscopy, surgical ablation, BCG instillation into the bladder, instillation of other chemotherapeutic molecules into the bladder, radiation treatment, or cystectomy should be performed or considered for performance. By performing the method on a recurring basis for a patient currently being treated for bladder cancer, such as by chemotherapy or radiation, the efficacy of the treatment can be monitored. By performing the method on a recurring basis for a patient previously treated for bladder cancer, the extent of recurrence, if any, can be monitored.

We have discovered the method has both high specificity (in the example below, with n = 81, the specificity was 1.0, meaning the false positive rate was 0%) and high sensitivity (in the example below, the sensitivity was 0.86, meaning the false negative rate was 14%). To our knowledge, no other workers have reported a method of noninvasive bladder cancer screening, tested on a sample size of at least 80 individuals, having both a specificity above 0.85 and a sensitivity above 0.85, let alone a specificity of 1.0 and a sensitivity of at least 0.86.

The following examples are included to demonstrate preferred embodiments of the invention.

### Example 1

### Summary

*Background*: Chromosomal missegregation resulting in aneuploidy is a common somatic genetic change in neoplasia, and genes regulating segregation of chromosomes may be potential cancer detection markers. Amplification/overexpression of aurora kinase A, a key regulator of mitosis, has been frequently detected in human cancer including bladder cancer and the degree of amplification correlates with the degree of aneuploidy.

*Methods*: We measured the level of aurora kinase A expression in bladder cancer cells and correlated it with the copy number of three selected chromosomes as well as total nuclear DNA content. The effect of aurora kinase A on centrosome multiplication and chromosome copy number was measured *in vitro* using an adenoviral expression construct. To assess the applicability of this gene as a biomarker for bladder cancer, we also tested the aurora kinase A gene copy number using fluorescence in situ hybridization (FISH) on exfoliated cells from voided urine of bladder cancer patients.

*Findings:* Overexpression of aurora kinase A in human urothelial cells induced amplification of centrosomes, chromosome missegregation, and aneuploidy. Moreover, overexpression of the aurora kinase A gene occurred in early phases of tumor development and could be detected in *in situ* lesions as well as in voided urine of patients with bladder cancer. A FISH test for aurora kinase A gene copy number performed on a blinded validation test consisting of 51 voided urine samples from patients with bladder cancer and 30 unaffected healthy controls detected bladder cancer with specificity 1.0 and sensitivity 0.86.

*Interpretation*: Our findings indicate that overexpressed aurora kinase A can cause aneuploidy in urothelial cells and is a promising biomarker for detection of bladder cancer.

### Introduction

Human bladder cancer is an ideal system to study the mechanisms of chromosomal instability as it develops from *in situ* preneoplastic conditions via papillary and non-papillary pathways, which show strong correlation between aggressiveness and the degree of aneuploidy.¹¹ Most low-grade, superficial papillary tumors that originate from urothelial hyperplasia are near diploid. Although they often recur, they are unlikely to invade the bladder wall and metastasize. By contrast, virtually all high-grade, nonpapillary tumors that develop by progression from severe dysplasialcarcinoma *in situ* show pronounced aneuploidy and have a high propensity to invade the bladder wall and metastasize.

In the present study, we evaluated the role of aurora kinase A in the development of bladder cancer from *in situ* urothelial neoplasia. We investigated the levels of aurora kinase A amplification and overexpression in human bladder tumor samples and bladder cancer cell lines and correlated them with the degree of aneuploidy. The functional implications of aurora kinase A overexpression were validated by *in vitro* transfection studies. Finally, we identified aurora kinase A as a potential biomarker for non-invasive detection of bladder cancer in urine.

### Materials and Methods

*Tumor samples and cell lines*. Cell suspensions from paired samples of transitional cell carcinoma (TCC) of the bladder and adjacent urothelium were obtained from 21 human cystectomy specimens as previously described.¹² Briefly, the mucosal surface adjacent to tumor was scraped and the cells were transferred into a conical tube containing phosphate buffered solution (PBS). The tumor was dissected from a frozen block to minimize contamination with non-tumor cells and similarly transferred to a conical tube containing PBS. Tumor cells were released into a PBS solution by mechanically agitating the tissue fragments. Only those samples that yielded greater than 90% of microscopically recognizable tumor cells or intact urothelium were utilized for this study. Voided urine specimens (approximately 100-200 ml) from 74 patients with cystoscopically evident bladder cancer were collected prior to transurethral resection of tumors and centrifuged for 15 minutes at 1000 RPI. The urine sediments were re-suspended in 2 ml of DMEM with 10% of DMSO and stored in -70°C. The intraurothelial precancerous changes were classified as mild, moderate, and severe dysplasia or carcinoma *in situ.* The tumors were classified according to the three-tier World Health Organization histological grading system and growth pattern (papillary versus non-papillary). The depth of invasion was recorded according to the TNM (tumor-node-metastasis) staging system. Voided urine specimens from 37 unaffected healthy individuals without evidence of bladder cancer served as controls. The human bladder cancer cell lines UM-UC-1, UM-UC-2, UM-UC3, UM-UC-6, UM-UC-9, UM-UC-10, UM-UC-12, UM-UC-13, UM-UC-15, UM-UC-16, and UM-UC-17, and normal human urothelial cells were established by us and the SV40 immortalized human urothelial cell line, SVHUC, was obtained from Dr. C. A. Reznikoff and cultured as previously described.¹³

*Expression assays for aurora kinase A*. Quantitative RT-PCR was used to analyze the expression levels of aurora kinase A in paired samples of adjacent urothelial/bladder tumors and cell lines as previously described.¹² Briefly, for RT-PCR, cDNA was synthesized from 2 µg of total RNA using the TaqMan RT reagents following the manufacturer's protocol (Applied Biosystems, Foster City, CA). The primers and fluorescent probes for each gene were designed according to the Assays-by-Design Service provided by Applied Biosystems. RT-PCR analysis was performed on a PerkinElmer/Applied Biosystems 7700 Prism apparatus, using housekeeping gene 18S as an internal normalization standard. Urothelium suspensions prepared from ureters from nephrectomy specimens free of urothelial neoplasia were used as standards from which we calculated the relative expressions of aurora kinase A from adjacent urothelium and bladder tumors. NU204 human urothelial cells were grown *in vitro* as previously described¹⁴ and were used as a reference to calculate relative expression levels of aurora kinase A in bladder cancer cell lines grown *in vitro.*

*Aurora kinase A gene copy number analysis.* A dual color aurora kinase A FISH probe (AURKA 20g13/a-satellite 20 DNA probe) purchased from MP Biomedicals/Qbiogene (Illkirch, France), was used to detect the copy number of the aurora kinase A gene in exfoliated cells of voided urine sediments. The AURKA 20813 probe was directly labeled with Rhodamine (red) and the chromosome 20 α-satellite probe was directly labeled with Fluorescein (green). The hybridization conditions were as per manufacturer's recommendations.¹⁵ In brief, the voided urine sediments were defrosted and washed three times in PBS followed by centrifugation. The cystospin preparations were fixed in methanol/acetic acid (3:1) and were pretreated in 2x SSC/0.5% NP-40, pH7 at 37°C for 30 minutes followed by dehydration in increasing gradients of ethanol. After denaturing at 90°C for 5 minutes the cells were hybridized overnight with 10 µl of AURKA 20q13 probe at 37°C. After they were washed for 5 minutes in 0.5x SSC/0.1% SDS for five minutes at 65°C, cells were counterstained with DAPI and mounted in an antifade solution. Fluorescence signals were counted and captured with Zeiss Axioplan 2 microscope.

*DNA ploidy analysis*. The ploidy of interphase nuclei was analyzed with centromeric FISH probes for chromosomes 3, 7, and 17 (Vysis Abbott; Park, IL) and by measurements of total nuclear DNA by image analysis with the SAMBA 4000 system (Ampersand Medical; Chicago, IL) as previously described.⁶ Normal human urothelial cells from resected nephrectomy specimens were used both to test the performance of FISH probes in normal human diploid tissue and to serve as a negative control.

DNA ploidy measurements were performed on cytospin preparations of paired samples of adjacent urothelium/bladder tumors and bladder cancer cell lines stained with the Feulgen reaction. The DNA index was calculated as the ratio of the mean nuclear DNA content of the tumor cells to the mean nuclear DNA content of a diploid standard (human peripheral blood lymphocytes). Tumors with DNA indices ranging from 0.9-1.2 were classified as diploid/near-diploid whereas tumors in which a DNA index of greater than 1.2 was in more than 20% of cells with a distinct peak on the histogram were classified as aneuploid.

*In vitro transfection assays. In vitro* transfection studies were carried out using pcDNA3-wild type aurora kinase A and simian virus 40 (SV40) immortalized human urothelial cells as previously described.⁴ Briefly, cells transfected with adenoviral GFP vector without aurora kinase A served as controls. Only those experiments that showed greater than or equal to 80% transfection rates were accepted for analysis. The degree of aurora kinase A-GFP ectopic expression was verified by western blot analysis using antiGFP antibodies and by RT-PCR using primers targeting the aurora kinase A-GFP fusion sequence. The cells were subsequently harvested on days 1-4 after transfection and used for: analysis of centrosome copy number, simultaneous flow cytometric measurements of total DNA content and aurora kinase A expression levels, chromosomal copy number with centromeric FISH probes for chromosomes 3, 7, and 17, and soft agar colony formation assay.

For centrosome copy number, methanol fixed cells were exposed to anti-y-tubulin mouse antibody (1:2000 dilution, Sigma Chemical Corp., St. Louis, MO) for 1 hour as previously described.⁴ Briefly, the bound primary antibody was detected with Texas Red-conjugated anti-mouse antibody (1:500 dilution, Vector Laboratories, Burlingame, CA) using DAPI as counterstain.

Simultaneous flow cytometric analysis of DNA content (red fluorescence of propidium iodide; PI) and expression levels of aurora kinase A (green fluorescence of GFP) was carried out using a fluorescence-activated cell sorter (EPICS XL-MCL, Beckman Coulter, Inc, Miami, FL.) The population of cells with abnoiinal DNA content was computed as the number of GFP positive cells with a DNA index < 2c and > 4c.

For soft agar colony formation assays, SV40 immortalized urothelial cells were infected with an adenovirus vector containing wild type aurora kinase A tagged with GFP or a control adenovirus containing GFP only. One day after viral infection, the cells were plated at a concentration of 10,000 cells/plate on soft agar containing UM-UC-3 conditioned medium. The number of colonies per plate was recorded two weeks after plating.

*Statistical Analysis*. For quantitative analysis of FISH both green and red signals were counted in at least 20 cells from each urine sample and the number of green and red signals for each cell was recorded. The cells containing abnormal aurora A signal were divided into two sub-groups, consisting of cells with low (3-4 copies) and high (>4 copies) levels of aurora kinase A gene amplification.. For each sample we calculated an aurora kinase A score by dividing the number of cells with elevated levels (3 or more copies) by the total number of cells examined. An aurora kinase A score >0.15 was considered to be elevated and corresponded to >3 cells out of a set of 20 with at least 3 red FISH signals. This score was used to produce ROC curves for a training set of seven controls and 23 bladder cancer urine samples (n = 30). The performance of the aurora kinase A FISH test was further validated on a testing set of 30 controls and 51 bladder cancer urine samples (n = 81). The area under the curve (AUC) was used to assess the performance of the marker. The AUC is equivalent to a Wilcoxon-Mann-Whitney rank sum test comparing the case and control groups,¹⁶ and the latter test was used to derive p-values. Chi-squared tests were used on the data from cancer samples to assess the association between aurora kinase A score and pathologic features of the tumors such as histologic grade, growth pattern, and stage. In cases where the contingency table counts were small, p-values were assessed by simulation with marginal counts fixed (p<--0.05 was considered significant).

### Results

We first investigated the expression levels of aurora kinase A in samples of TCC of the bladder and its adjacent *in situ* preneoplastic urothelium. The levels of aurora kinase A mRNA revealed by quantitative RT-PCR were elevated in both adjacent urothelium and TCC (Fig. 1A). The expression levels of aurora kinase A correlated with tumor grade, stage, and aneuploidy (Fig. 1B-D). Low-grade, superficial TCCs that showed minimal deviation from normal diploid of their total nuclear DNA content and were classified as near diploid showed mild elevation of aurora kinase A expression (approximately 2-fold) as compared to normal urothelium. In contrast, high-grade, invasive TCCs with pronounced aneuploidy showed marked elevation of aurora kinase A expression (approximately 7-fold).

To determine whether the degree of chromosomal instability was related to the degree of overexpression of aurora kinase A, we analyzed aurora kinase A expression levels and chromosome copy number in bladder cancer cell lines (Fig. 2A,B). Using expression levels of aurora kinase A, we divided bladder cancer cell lines into three groups (Fig. 2C). The first group consisted of 6 cell lines with no elevation of aurora kinase A expression. The second group consisted of 4 cell lines with mild aurora kinase A elevation (2- to 5-fold). The remaining 3 cell lines showed markedly elevated levels of aurora kinase A (6- to 14-fold). The analysis of chromosomal copy numbers using centromeric FISH probes for selected chromosomes (3, 7, 17) showed dramatically increased numbers of chromosomes in cell lines with markedly elevated levels of aurora kinase A (Fig. 2D).

These correlative studies suggested that gain of function of aurora kinase A through amplification and overexpression gives rise to aneuploid cellular phenotypes during bladder cancer development. We verified this hypothesis by *in vitro* transfection studies using SV40-immortalized urothelial cells and adenoviral constructs containing wild-type aurora kinase A-GFP inserts. The transfected urothelial cells showed a marked elevation of ectopically expressed aurora kinase A that was associated with amplification of centrosomes and development of aneuploidy as well as transformed cellular phenotype revealed by the presence of increased number of centrosome signals, chromosomal copy number, total DNA content, and acquisition of colony forming ability on soft agar (Fig. 3A-D).

To determine whether aurora kinase A can be used as a marker for bladder cancer detection, we analyzed voided urine sediments of patients with bladder cancer for amplification of aurora kinase A using the FISH technique (Fig. 4A). The analysis was performed in a blinded fashion; i.e., the observer scoring the FISH results did not know whether the sample was obtained from a patient with bladder cancer or an unaffected control. The initial testing was performed on training set of voided urine sediments from 23 patients with bladder cancer and seven healthy controls. It revealed at least low-levels of gene amplification (3-4 copies) in all 23 patients with bladder cancer. The voided urine sediments from patients with low-grade TCC contained primarily cells that had 3-4 copies of aurora kinase A while a substantial proportion of cells (>20%) from patients with high-grade TCC had >4 copies of aurora kinase A (Fig. 4B). Two cells with three copies of aurora kinase A signal were detected in one control. No abnormal copy numbers of aurora kinase A were detected in urine sediments of the remaining six controls. The analysis of data from the training set using >15% of cells with at least 3 copies of aurora kinase A as the cut-off for the positivity of the test yielded a specificity of 1.0, a sensitivity of 0.91, and an ROC AUC of 0.997 (p = 1 x 10⁻⁴).

We validated the performance of the aurora kinase A FISH test for bladder cancer on an additional blinded testing set consisting of voided urine samples from 51 bladder cancer patients and 30 healthy controls. The data were analyzed using the same cut-off point for the positivity of the test i.e. >15% of cells with at least 3 copies of aurora kinase A. The test was positive in 44 samples of patients with bladder cancer. No abnormal aurora kinase A gene copy numbers were identified in seven patients with bladder cancer. In five out of 30 control samples, 1-3 cells with three copies of the aurora kinase A gene were identified. The analysis of the testing set yielded a specificity of 1.0, a sensitivity of 0.84, and an ROC AUC of 0.925 (p = 6.79 x 10⁻¹¹). The results of quantitative FISH analysis of cells in voided urine for all 74 patients with bladder cancer and 37 healthy controls are summarized in Figure 5. The degree of aurora kinase A amplification as measured by the aurora score strongly correlated with the histologic grade of the tumors. Bladder cancers of high histologic grade (grade 3) had significantly higher aurora kinase A scores (0.60 ± 0.29) than low grade (grade 1-2) tumors (0.13 ± 0.15). There was no significant correlation between the score and the tumor growth pattern (papillary vs. nonpapillary), or the stage (superficial vs. invasive) (data not shown).

### Discussion

The current findings in light of those published earlier provide strong evidence in favor of aurora kinase A amplification lover expression being an early event in bladder carcinogenesis that is associated with the development of centrosome amplification and aneuploid genomic content. These observations assume important biological significance in view of our earlier reported findings that aurora kinase A facilitates degradation of tumor suppressor protein p53 and that human bladder tumor samples with elevated expression of aurora kinase A reveal extremely low cellular p53 content, mimicking loss of a critical tumor suppressor pathway.¹⁷

It is relevant in this context to mention that loss or inactivating mutation of certain tumor suppressor proteins, most notably p53, causes centrosome amplification¹⁸ and that concomitant occurrence of p53 mutation and cyclin E over-expression is strongly associated with chromosome instability and centrosome amplification in bladder cancer.¹⁹ The recent discovery of an interaction and feedback regulation between aurora kinase A and the focal adhesion scaffolding protein HEF1 indicates that elevated expression of aurora kinase A may coordinately derange the integrin dependent signaling processes controlling cell attachment, migration, and cell survival along with induction of chromosomal instability manifested as aneuploidy.²⁰ These observations not only provide strong evidence for a critical role of aurora kinase A in malignant transformation of urothelial cells, but also suggests that it may be a novel biomarker for bladder cancer.

Collectively, our data indicates that aurora kinase A when overexpressed in urothelial cells causes centrosome amplification and missegregation of chromosomes, resulting in aneuploidy and transformed phenotypes. Current FISH studies on voided urine samples of patients with bladder cancer together with the previously published data indicate that overexpression and amplification of aurora kinase A is frequent in bladder cancer and can be detected in exfoliated urothelial cells from voided urine sediment. The dual-color FISH probe for aurora kinase A gene and α-satellite centromeric chromosome 20 DNA detected bladder cancer with high specificity and sensitivity. Moreover, the degree of aurora kinase A amplification correlated with high grade clinically aggressive bladder cancer. This implies that testing for this aurora kinase copy number by FISH technique in urine is a promising noninvasive test for bladder cancer.

Further, to the best of our knowledge, our work is the first to move beyond correlative studies *suggesting* that gain of function of aurora kinase A through amplification and overexpression gives rise to aneuploid cellular phenotypes during bladder cancer development to show that overexpression of aurora kinase A in urothelial cells *causes* centrosome amplification and missegregation of chromosomes, resulting in aneuploidy and transformed phenotypes.

All of the methods disclosed and claimed herein can be made and executed without undue experimentation in light of the present disclosure.

### SEQUENCE LISTING

<110> Grossman, H. Barton Czerniak, Bogdan
<120> Noninvasive Detection of Bladder Cancer by Fluorescence In Situ Hybridization of Aurora Kinase A
<130> MDA07-125, 4002.001790
<160> 2
<170> PatentIn version 3.3
<210> 1
   <211> 2128
   <212> DNA
   <213> Homo sapiens
<400> 1
   cttggaagac ttgggtcctt gggtcgcagg ctggagtgca atggtgtgat ctcagctcac      60
   tgcaacctct gcttcctggg tttaagtgat tctcctgcct cagcctcccg agtagctggg     120
   attacaggca tcatggaccg atctaaagaa aactgcattt caggacctgt taaggctaca     180
   gctccagttg gaggtccaaa acgtgttctc gtgactcagc aatttccttg tcagaatcca     240
   ttacctgtaa atagtggcca ggctcagcgg gtcttgtgtc cttcaaattc ttcccagcgc     300
   gttcctttgc aagcacaaaa gcttgtctcc agtcacaagc cggttcagaa tcagaagcag     360
   aagcaattgc aggcaaccag tgtacctcat cctgtctcca ggccactgaa taacacccaa     420
   aagagcaagc agcccctgcc atcggcacct gaaaataatc ctgaggagga actggcatca     480
   aaacagaaaa atgaagaatc aaaaaagagg cagtgggctt tggaagactt tgaaattggt     540
   cgccctctgg gtaaaggaaa gtttggtaat gtttatttgg caagagaaaa gcaaagcaag     600
   tttattctgg ctcttaaagt gttatttaaa gctcagctgg agaaagccgg agtggagcat     660
   cagctcagaa gagaagtaga aatacagtcc caccttcggc atcctaatat tcttagactg     720
   tatggttatt tccatgatgc taccagagtc tacctaattc tggaatatgc accacttgga     780
   acagtttata gagaacttca gaaactttca aagtttgatg agcagagaac tgctacttat     840
   ataacagaat tggcaaatgc cctgtcttac tgtcattcga agagagttat tcatagagac     900
   attaagccag agaacttact tcttggatca gctggagagc ttaaaattgc agattttggg     960
   tggtcagtac atgctccatc ttccaggagg accactctct gtggcaccct ggactacctg    1020
   ccccctgaaa tgattgaagg tcggatgcat gatgagaagg tggatctctg gagccttgga    1080
   gttctttgct atgaattttt agttgggaag cctccttttg aggcaaacac ataccaagag    1140
   acctacaaaa gaatatcacg ggttgaattc acattccctg actttgtaac agagggagcc    1200
   agggacctca tttcaagact gttgaagcat aatcccagcc agaggccaat gctcagagaa    1260
   gtacttgaac acccctggat cacagcaaat tcatcaaaac catcaaattg ccaaaacaaa    1320
   gaatcagcta gcaaacagtc ttaggaatcg tgcaggggga gaaatccttg agccagggct    1380
   gccatataac ctgacaggaa catgctactg aagtttattt taccattgac tgctgccctc 1440
   aatctagaac gctacacaag aaatatttgt tttactcagc aggtgtgcct taacctccct 1500
   attcagaaag ctccacatca ataaacatga cactctgaag tgaaagtagc cacgagaatt 1560
   gtgctactta tactggttca taatctggag gcaaggttcg actgcagccg ccccgtcagc 1620
   ctgtgctagg catggtgtct tcacaggagg caaatccaga gcctggctgt ggggaaagtg 1680
   accactctgc cctgaccccg atcagttaag gagctgtgca ataaccttcc tagtacctga 1740
   gtgagtgtgt aacttattgg gttggcgaag cctggtaaag ctgttggaat gagtatgtga 1800
   ttctttttaa gtatgaaaat aaagatatat gtacagactt gtattttttc tctggtggca 1860
   ttcctttagg aatgctgtgt gtctgtccgg caccccggta ggcctgattg ggtttctagt 1920
   cctccttaac cacttatctc ccatatgaga gtgtgaaaaa taggaacacg tgctctacct 1980
   ccatttaggg atttgcttgg gatacagaag aggccatgtg tctcagagct gttaagggct 2040
   tattttttta aaacattgga gtcatagcat gtgtgtaaac tttaaatatg caaataaata 2100
   agtatctatg tcaaaaaaaa aaaaaaaa 2128
<210> 2
   <211> 2112
   <212> DNA
   <213> Homo sapiens
<400> 2
   cttggaagac ttgggtcctt gggtcgcagg ctggagtgca atggtgtgat ctcagctcac     60
   tgcaacctct gcttcctggg tttaagtgat tctcctgcct cagcctcccg agtagctggg    120
   attacaggca tcatggaccg atctaaagaa aactgcattt caggacctgt taaggctaca    180
   gctccagttg gaggtccaaa acgtgttctc gtgactcagc aatttccttg tcagaatcca    240
   ttacctgtaa atagtggcca ggctcagcgg gtcttgtgtc cttcaaattc ttcccagcgc    300
   attcctttgc aagcacaaaa gcttgtctcc agtcacaagc cggttcagaa tcagaagcag    360
   aagcaattgc aggcaaccag tgtacctcat cctgtctcca ggccactgaa taacacccaa    420
   aagagcaagc agcccctgcc atcggcacct gaaaataatc ctgaggagga actggcatca    480
   aaacagaaaa atgaagaatc aaaaaagagg cagtgggctt tggaagactt tgaaattggt    540
   cgccctctgg gtaaaggaaa gtttggtaat gtttatttgg caagagaaaa gcaaagcaag    600
   tttattctgg ctcttaaagt gttatttaaa gctcagctgg agaaagccgg agtggagcat    660
   cagctcagaa gagaagtaga aatacagtcc caccttcggc atcctaatat tcttagactg    720
   tatggttatt tccatgatgc taccagagtc tacctaattc tggaatatgc accacttgga    780
   acagtttata gagaacttca gaaactttca aagtttgatg agcagagaac tgctacttat    840
   ataacagaat tggcaaatgc cctgtcttac tgtcattcga agagagttat tcatagagac    900
   attaagccag agaacttact tcttggatca gctggagagc ttaaaattgc agattttggg    960
   tggtcagtac atgctccatc ttccaggagg accactctct gtggcaccct ggactacctg   1020
   ccccctgaaa tgattgaagg tcggatgcat gatgagaagg tggatctctg gagccttgga   1080
   gttctttgct atgaattttt agttgggaag cctccttttg aggcaaacac ataccaagag   1140
   acctacaaaa gaatatcacg ggttgaattc acattccctg actttgtaac agagggagcc   1200
   agggacctca tttcaagact gttgaagcat aatcccagcc agaggccaat gctcagagaa   1260
   gtacttgaac acccctggat cacagcaaat tcatcaaaac catcaaattg ccaaaacaaa   1320
   gaatcagcta gcaaacagtc ttaggaatcg tgcaggggga gaaatccttg agccagggct   1380
   gccatataac ctgacaggaa catgctactg aagtttattt taccattgac tgctgccctc   1440
   aatctagaac gctacacaag aaatatttgt tttactcagc aggtgtgcct taacctccct   1500
   attcagaaag ctccacatca ataaacatga cactctgaag tgaaagtagc cacgagaatt   1560
   gtgctactta tactggttca taatctggag gcaaggttcg actgcagccg ccccgtcagc   1620
   ctgtgctagg catggtgtct tcacaggagg caaatccaga gcctggctgt ggggaaagtg   1680
   accactctgc cctgaccccg atcagttaag gagctgtgca ataaccttcc tagtacctga   1740
   gtgagtgtgt aacttattgg gttggcgaag cctggtaaag ctgttggaat gagtatgtga   1800
   ttctttttaa gtatgaaaat aaagatatat gtacagactt gtattttttc tctggtggca   1860
   ttcctttagg aatgctgtgt gtctgtccgg caccccggta ggcctgattg ggtttctagt   1920
   cctccttaac cacttatctc ccatatgaga gtgtgaaaaa taggaacacg tgctctacct   1980
   ccatttaggg atttgcttgg gatacagaag aggccatgtg tctcagagct gttaagggct   2040
   tattttttta aaacattgga gtcatagcat gtgtgtaaac tttaaatatg caaataaata   2100
   agtatctatg tc                                                       2112

### REFERENCES

1. Perkins AS, Stem DF. Molecular biology of cancer: oncogenes. In: Cancer: Principles and practices of oncology. Philadelphia: Lippincott-Raven Publishers, 5th edition, 1997: 79-119.
2. Heim S, Mitelman F. Cancer cytogenetics, 2nd edition. New York, 1995.
3. Sen S. Aneuploidy and cancer. In: Lenganer C ed. Current Opinion in Oncology, Lippincott, Williams, and Wilkins, 2000: Vol. 12, pp. 82-88.
4. Zhou H, Kuang J, Zhong L, et al. Tumour amplified kinase STK151BTAK induces centrosorne amplification, aneuploidy and transformation. Nat Genet 1998; 20: 189-93.
5. Katayama H, Zhou H, Li Q, et al. Interaction and feedback regulation between STK151BTAKIAurora-A kinase and protein phosphatase 1 through mitotic cell division cycle. JBiol Chem 2001; 276: 46219-24.
6. Sen S, Zhou H, Zhang RD, et al. Amplification/overexpression of a mitotic kinase gene in human bladder cancer. JNatl Cancer Inst 2002; 94: 1320-29.
7. Andrews PD. Aurora kinases: shining lights on the therapeutic horizon? Oncogene 2005; 24: 5005-15.
8. Bischoff JR, Anderson L, Zhu Y, et al. A homologue of Drosophila aurora kinase is oncogenic and amplified in human colorectal cancers. Embo J 1998; 17: 305265.
9. Savelieva E, Belair CD, Newton MA, et al. 20q gain associates with immortalization: 20q13.2 amplification correlates with genome instability in human papillomavirus 16 E7 transformed human uroepithelial cells. Oncogene 1997; 14: 551-60.
10. Ewart-Toland A, Briassouli P, de Koning JP, et al. Identification of Stk6/STK15 as a candidate low-penetrance tumor-susceptibility gene in mouse and human. Nat Genet 2003; 34: 403-12.
11. Spiess PE, Czerniak B. Dual track concept of bladder carcinogenesis: Practical implications. Arch Pathol Lab Invest 2006; 130:844-52.
12. Kim JH, Tuziak T, Hu L, et al. Alterations in transcription clusters underlie development of bladder cancer along papillary and nonpapillary pathways. Lab Invest 2005; 85: 532-49.
13. Christian BJ, Loretz LJ, Oberley TD, Reznikoff CA. Characterization of human uroepithelial cells immortalized in vitro by simian virus 40. Cancer Res 1987; 47: 6066-73.
14. Liebert M, Wedemeyer G, Chang JH, et al. Comparison of antigen expression on normal urothelial cells in tissue section and tissue culture. J Urol 1990; 144: 1288-92.
15. Sakakura C, Hagiwara A, Yasuoka R, et al. Tumour-amplified kinase BTAK is amplified and overexpressed in gastric cancers with possible involvement in aneuploid formation. Br J Cancer 2001; 84:824-31.
16. Hanley JA, McNeil BJ. The meaning and use of the area under a receiver operating characteristic (ROC) curve. Radiology 1982;143:29-36.
*17*. Katayama H, Sasai K, Kawai H, et al. Phosphorylation by aurora kinase A induces Mdm2-mediated destabilization and inhibition of p53. Nat Genet 2004; 36: 55-62*.*
18. Fukasawa K. Centrosome amplification, chromosome instability and cancer development. Cancer Lett 2005; 230: 6-19.
19. Kawamura K, Izumi H, Ma Z, et al. Induction of centrosome amplification and chromosome instability in human bladder cancer cells by p53 mutation and cyclin E overexpression. Cancer Res 2004; 64: 4800-09.
*20.* Pugacheva EN, Golemis EA. The focal adhesion scaffolding protein HEF1 regulates activation of the Aurora-A and Nek2 kinases at the centrosome. Nat Cell Biol 2005; 7: 937-46*.*

## Claims

1. A method of detecting bladder cancer in a patient, comprising: isolating bladder cells from the patient's urine; hybridizing the bladder cells with a labelled DNA probe, wherein the probe recognizes at least a portion of the aurora kinase A gene, to yield a sample of bladder cells hybridized with the labelled DNA probe; and counting the number of bladder cells in the sample, the number of copies of the aurora kinase A gene in each bladder cell in the sample, and the number of bladder cells in the sample having at least a first threshold number of copies of the aurora kinase A gene, further comprising, if greater than or equal to a first threshold percentage of the bladder cells have at least the first threshold number of copies of the aurora kinase A gene, presuming the patient to have bladder cancer, wherein the first threshold number of copies of the aurora kinase A gene is three, and wherein the first threshold percentage is 15%.

2. The method of claim 1, wherein counting further comprises counting the number of bladder cells having at least a second threshold number of copies of the aurora kinase A gene, wherein the second threshold number of copies is greater than the first threshold number of copies.

3. The method of claim 2, wherein the second threshold number of copies of the aurora kinase A gene is five.

4. The method of claim 1, further comprising, if greater than or equal to a second threshold percentage of the bladder cells have at least the first threshold number of copies of the aurora kinase A gene, wherein the second threshold percentage is greater than the first threshold percentage, presuming the patient to have aggressive bladder cancer.

5. The method of claim 4, wherein the second threshold percentage is 20%.

6. The method of claim 1, wherein the aurora kinase A gene comprises a DNA sequence selected from the group consisting of SEQ ID NO: 1 and SEQ NO:2.

## Patentansprüche

1. Verfahren zum Nachweis von Blasenkrebs in einem Patienten, umfassend: Isolieren von Blasenzellen aus dem Urin des Patienten; Hybridisieren der Blasenzellen mit einer markierten DNA-Sonde, wobei die Sonde zumindest einen Bereich des Aurora-Kinase-A-Gens erkennt, um eine Probe von Blasenzellen, die mit der markierten DNA-Sonde hybridisiert sind, zu ergeben; und Zählen der Anzahl von Blasenzellen in der Probe, wobei die Zahl der Kopien des Aurora-Kinase-A-Gens in jeder Blasenzelle in der Probe und die Zahl von Blasenzellen in der Probe zumindest eine erste Schwellenzahl von Kopien des Aurora-Kinase-A-Gens aufweisen, ferner mit, wenn mehr als oder gleich viel wie eine erste Schwellenprozentzahl der Blasenzellen zumindest die erste Schwellenzahl von Kopien des Aurora-Kinase-A-Gens aufweisen, Annehmen, dass der Patient Blasenkrebs hat, wobei die erste Schwellenzahl von Kopien des Aurora-Kinase-A-Gens drei beträgt und wobei die erste Schwellenprozentzahl 15% beträgt.

2. Verfahren nach Anspruch 1, wobei das Zählen ferner das Zählen der Anzahl von Blasenzellen mit zumindest einer zweiten Schwellenzahl von Kopien des Aurora-Kinase-A-Gens umfasst, wobei die zweite Schwellenzahl von Kopien größer als die erste Schwellenzahl von Kopien ist.

3. Verfahren nach Anspruch 2, wobei die zweite Schwellenzahl von Kopien des Aurora-Kinase-A-Gens fünf ist.

4. Verfahren nach Anspruch 1, ferner mit, wenn mehr als oder gleich viel wie eine zweite Schwellenprozentzahl der Blasenzellen zumindest die erste Schwellenzahl von Kopien des Aurora-Kinase-A-Gens aufweisen, wobei die zweite Schwellenprozentzahl größer als die erste Schwellenprozentzahl ist, Annehmen, dass der Patient aggressiven Blasenkrebs hat.

5. Verfahren nach Anspruch 4, wobei die zweite Schwellenprozentzahl 20% beträgt.

6. Verfahren nach Anspruch 1, wobei das Aurora-Kinase-A-Gen eine DNA-Sequenz umfasst, die aus der Gruppe ausgewählt ist, die aus SEQ ID NO:1 und SEQ ID NO:2 besteht.

## Revendications

1. Un procédé de détection du cancer de la vessie chez un patient, comprenant l'isolement de cellules de la vessie à partir de l'urine du patient ; l'hybridation des cellules de la vessie avec une sonde ADN marquée, ladite sonde identifiant au moins une partie du gène kinase Aurora A, pour obtenir un échantillon des cellules de la vessie hybridées avec la sonde ADN marquée ; et le décompte du nombre de cellules de la vessie dans l'échantillon, du nombre de copies du gène kinase Aurora A dans chaque cellule de la vessie dans l'échantillon, et du nombre de cellules de la vessie dans l'échantillon ayant au moins un premier nombre seuil de copies du gène kinase Aurora A, supposant également, si un nombre supérieur ou égal à un premier pourcentage seuil de cellules de la vessie possède au moins le premier nombre seuil de copies du gène kinase Aurora A, de supposer que le patient a le cancer de la vessie, le premier nombre seuil de copies du gène kinase Aurora A étant de trois et le premier pourcentage seuil étant de 15 %.

2. Le procédé de la revendication 1, dans lequel le décompte suppose également de compter le nombre de cellules de la vessie qui possède au moins un deuxième nombre seuil de copies du gène kinase Aurora A, dans lequel le deuxième nombre seuil de copies est supérieur au premier nombre seuil de copies.

3. Le procédé de la revendication 2, dans lequel le deuxième nombre seuil de copies du gène kinase Aurora A est de cinq.

4. Le procédé de la revendication 1, supposant également, si un nombre supérieur ou égal à un deuxième pourcentage seuil de cellules de la vessie possède au moins le premier nombre seuil de copies du gène kinase Aurora A, le deuxième pourcentage seuil étant supérieur au premier pourcentage seuil, de supposer que le patient a un cancer de la vessie agressif.

5. Le procédé de la revendication 4, dans lequel le deuxième pourcentage seuil est de 20 %.

6. Le procédé de la revendication 1, dans lequel le gène kinase Aurora A comprend une séquence d'ADN sélectionnée dans le groupe se composant de SEQ ED NO: 1 et SEQ ID NO: 2.
